# EUROPEAN PATENT APPLICATION

(11) **EP 4 559 470 A1**
(43) Date of publication of application: **28.05.2025**
(21) Application number: 23211345.6
(22) Date of filing: 22.11.2023
(51) Int. Cl.: A61K 35/742, A61P 37/04, C12N 1/20

(54) **USE OF BACILLUS SUBTILIS LYSATE FOR THE IMMUNOMODULATION OF THE RESPIRATORY SYSTEM CELLS**

(71) Applicant: State Research Institute Centre for Innovative Medicine, 08406 Vilnius (LT)
(72) Inventor: Aldonyte, Ruta, LT-08406 Vilnius (LT); Bironaite, Daiva, LT-08406 Vilnius (LT); Bagdonas, Edvardas, LT-08406 Vilnius (LT); Raudoniute, Jovile, LT-08406 Vilnius (LT); Konovalovas, Aleksandras, LT-08406 Vilnius (LT); Danila, Edvardas, LT-08406 Vilnius (LT); Armalyte, Julija, LT-08406 Vilnius (LT)

(57) **Abstract**

The invention discloses a method and an application of the lysate of Bacillus subtilis for the stimulation of human bronchial epithelium and monocytes *in vitro.* This invention has direct relevance as an immune stimulator administered against infectious bacterial and non-bacterial diseases when an augmented immune response against an unknown pathogen is required. It works on innate immunity cells, i.e. antigen-presenting monocyte-derived-macrophages, and within airway epithelium by enhancing barrier functions and promoting the release of inflammatory mediators. *In vitro* effects of the lysate involve monocyte differentiation towards macrophages and stimulation of the epithelium to release cytokines and chemokines and tightened intraepithelial junctions. The method proposed here offers an efficient, physiological and affordable way to induce immune stimulation. Such method may be applied in vitro and in clinic since Bacillus subtilis is a ubiquitous bacterium present in water, soil, air, and is considered a beneficial microorganism as it does not possess traits that cause disease. It is not considered pathogenic or toxigenic to humans, animals, or plants.

## Description

### APPLICATIONS

### FIELD OF THE INVENTION

We hereby report a bacterial lysate with immunomodulatory and immunostimulatory effects on human bronchial epithelium to be used in experiments *in vitro* and clinically to modulate inflammatory activation of human airway epithelium and monocytes. The invention should be used for human and animal upper airway infections via mucosal delivery or as a stimulator of bronchial epithelium and monocytes *in vitro.*

### BACKGROUND OF THE INVENTION

Immunomodulation is a regulation and modulation of immune response. It can be achieved by enhancing or suppressing of the activity of the immune system, depending on the condition being treated as therapeutic or preventive. Immunomodulatory agents include various synthetic medicines, vaccines, and biological compounds.

Immunostimulation refers to the enhancement of the immune response. It can be achieved through the use of immunostimulants, i.e., substances that are capable of activating and boosting the immune system. Described and proven examples of immunomodulatory and immunostimulatory substances include:
- corticosteroids (immunomodulators): these compounds suppress the immune system and are used to ameliorate the over-reactive immune system in cases of autoimmune diseases, allergies, and dysregulated inflammatory conditions.
- biologics (immunomodulators): genetically engineered proteins and similar substances that target specific molecules involved in the immune response. Examples include TNF inhibitors, which are used to treat rheumatoid arthritis and other autoimmune diseases.
- interferons (immunostimulatory) - proteins that enhance the immune response by stimulating the adaptive immune response, i.e. production of specific immune cells. They are used to treat viral infections and some types of cancer.
- vaccines (immunostimulatory) stimulate the immune system to produce an immune response to a specific pathogen.
- bacterial proteins utilize various pathways to modulate human immune system via signaling and interference with the transcription and translation of host proteins (1).

Bacillus subtilis is one of those bacteria that are abundant and prevalent virtually everywhere. Bacillus subtilis exhibits quite divergent effects on human systems and on local microbial communities. It is a gram-positive, spore-forming bacterium that is prevalent in the air, water, and soil and also in the gastrointestinal tract of humans and animals. There is a growing interest in exploring the potential health benefits of B. subtilis, and some studies have already shown that beneficial immunomodulatory effects on human cells might be expected. Historically B. subtilis was used widely for intestinal infection (diarrhea) (2). Also, recently, it was shown to work as a preventive medicine for viral airway infection, particularly caused by respiratory syncytial virus (RSV) (3).

Here are some scientifically described effects of Bacillus subtilis on prokaryotic and eukaryotic cells and macro-organisms :
- activation and proliferation of immune cells, i.e. macrophages, , and upregulated production of pro-inflammatory cytokines that regulate the inflammatory process and immune processes (4).
- enhancement and restoration of intestinal microbiota and improvement of gut health by promoting the growth of beneficial gut bacteria and inhibiting the growth of harmful bacteria. When supplemented as a probiotic, Bacillus subtilis also improves gut barrier function, subsequently reducing inflammation and all associated clinical symptoms (5).
- B. subtilis produces a range of antimicrobial compounds, e.g., antibiotics and bacteriocins, which can inhibit the growth of other bacteria and fungi (6).
- B. subtilis-secreted menaquinone-7 reduces symptoms of osteoporosis (7) and the risk of coronary heart disease (8).
- proteases secreted by B. subtilis are used in food industries (9, 10).
- B. subtilis products may become substitutes for antibiotics to treat infection by Clostridium perfringens (11).
- In addition, B. subtilis may serve as a biopesticide for fungi due to the production of antifungal lipopeptides (12).
- Lipopeptides, i.e. surfactins, mycosubtilins, and plipastatins, consist of fatty acid and peptide chain and can act as biosurfactants and antimicrobials (13).
- Anti-adhesive properties of B. subtilis secreted lipopeptide were also demonstrated, supporting B. subtilis uses where anti-biofilm activity is needed (14).
- B. subtilis also secretes surfactins, which exhibit antitumor, antiviral, anti-legionella, and antiplatelet aggregation actions (15-18).
- Lipopeptides derived from B. subtilis are used as emulsifying, anti-biofilm, and organoleptically improving agents (19).
- In the cosmetic industry B. subtilis secreted lipopeptides are used for their wound healing enhancement (20) and anti-wrinkle/moisturizing properties (21).

Mechanisms by which B. subtilis produced lipopeptides act are partially known. For instance, antioxidant effects or capacity to mitigate the oxidation of structural tissues, by B. subtilis products results from direct scavenging of ROS, particularly where local ROS production is not sufficiently counterbalanced by local antioxidants. Another mechanism is the activation of early signaling pathways and upregulation of expression of defense genes.

The effects of B. subtilis on human cells are not studied enough and more research is needed to fully understand the mechanisms involved and to determine the effects, optimal dose, method and duration of B. subtilis supplementation.

Many patents cover the use of B. subtilis. Listed below are several most relevant ones.
1. US Patent 11,071,101 describes a method for producing B. subtilis strain capable of increasing cytokine production. This document showed an increased production of cytokines and subsequently, upregulated immune responses. B. subtilis was suggested for use as a probiotic.
2. US Patent 10,976,926 contains documentation on producing B. subtilis strain capable of improving gut barrier function. This patent describes how improved gut barrier function may help to reduce inflammation and improve overall gut health. The patented strain is intended to use as a probiotic too.
3. US Patent 10,736,657 addresses composition comprising B. subtilis for improving animal growth performance and reducing pathogen load in the gut. The composition is intended for use as a feed additive for livestock.
4. US Patent 10,507,321 - "Method of enhancing Bacillus subtilis spore germination": This patent describes a method for enhancing the germination of Bacillus subtilis spores, which can improve the effectiveness of Bacillus subtilis as a probiotic or in the production of a food or feed product.
5. US Patent 10,335,386 describes B. subtilis strains that promote plant growth by reducing pathogen load in the soil. The strains are intended to use as biocontrol agents in agriculture.

These patents are just a few examples of the many patents related to Bacillus subtilis that are currently available.

Increasingly more preventive and supportive therapies for respiratory tract infection have been proposed. Probiotics are considered as one of the promising and safe candidates (22). The probiotics produce secondary substances that inhibit pathogen growth, stimulate the immune system and directly replace the pathogens (23-25). Recent studies and clinical trials have shown that in addition to the digestive tract where they are targeting, certain probiotics also have collateral effects in the respiratory tract (26, 27). Other upper airway-related effects include the reduced incidence of ear infections and less frequent need for antibiotics (28).

However, these effects were induced by digestive tract-applied probiotics, while applications within the respiratory system are unknown. Oral administrative probiotics and prebiotics, as food products, might be active in the upper airways too, and mitigate infection symptoms to some extent (29, 30).

Live Bacillus preparations might be unstable in suspension and are often used orally in a dry form. Therefore, we explored the effectiveness of lysed, not viable, and not multiplying formulation containing Bacillus subtilis. Such formulation might be applied in the respiratory tract as a spray or similar. Other application methods are also possible, including skin, genitourinary tract, and others. Here, we describe a liquid preparation of lysed Bacillus subtilis applicable as a prebiotic and effective immunomodulating agent and a macrophage maturation-inducing agent in vitro. The formulation contained 10⁸/mL of B. *subtilis* in 0.9% saline suspension.

### SUMMARY OF INVENTION

*Technical problem.* The background of the problem this current proposal addresses is that B. subtilis lysate, i.e. inactivated form of bacterial suspension, was never tested in airway epithelium, and its' possible prebiotic and postbiotic effects in the airways are not known. Inactivated bacterial preparation is stable and safe to use in the airways and elsewhere where dry preparations can not be applied. As presented in the state-of-the-art section above, the beneficial effects of B. subtilis are well described and widely employed in various fields, including clinical use, the food industry, agronomy, and veterinary. The potential of the products from B. subtilis to modulate airway inflammation and affect structural and immune cells in the human respiratory tract remains to be investigated. This will open several novel methods for using B. subtilis products clinically to manage certain pathologies of the human respiratory system.

*Solution.* This invention provides a method of how to safely and effectively employ bacterial products (B. subtilis lysate) on the bronchial epithelium and primary monocytes. We have documented certain immunomodulatory and epithelial barrier-enhancing properties of B. subtilis lysate and also investigated its effects on human monocytes *in vitro.* The latter resulted in monocyte maturation and macrophage phenotype induction. The invention extends the use of B. subtilis products (spores, live bacteria, and isolated substances, like lipopeptides) from the gastrointestinal tract to the respiratory tract and describes a novel *in vitro* method for macrophage activation and maturation.

Effects were tested *in vitro* by employing human bronchial epithelium and human monocyte lines. Relevant concentration and exposure methods were chosen for the B. subtilis lysate. Assessment of the effects included cell viability and proliferation measurements, barrier protein levels, namely occludin, and measurements of secreted pro-inflammatory mediators via enzyme-linked immunosorbent assay (ELISA). Monocytes were exposed to increasing doses of B. subtilis lysate and to phorbol 12-myristate 13-acetate (PMA) as a positive control. After 48-72 hours, their morphology (cell shape and size) and secretory content (pro-inflammatory molecules) were evaluated. B. subtilis-induced macrophage phenotype was described and compared to the PMA-induced phenotype. Macrophage phenotype was well developed and pronounced in B. subtilis-treated cells, and the suitability of the lysate for use *in vitro* for the development of monocyte-derived-macrophage was demonstrated.

*Advantageous effects.* The invention presents an improved method for the modulation of the inflammatory properties of human bronchial epithelium and for the *in vitro* derivation of macrophages from the monocytes. This is possible to achieve by employing safe and efficient concentrations of the abundant beneficial microorganism B. subtilis as described hereby. The developed lysate induces (a) an immunomodulatory effect, or (b) an immune potentiation effect, (c) a macrophage maturation effect, or (d) any combination of two effects (a) to (c). The invention uses lysate of B. subtilis, which contains the entire DNA, proteins and other bacterial content. The lysate is simple to produce and does not require purification or cold chain storage. It is suitable for incorporation into pharmaceutical compositions for administration as suspensions for mucosal delivery in nasal, nasopharyngeal or inhalation delivery. The dose at which the substance of the invention is applied *in vitro* or administered to a patient or to an animal will depend upon a variety of factors such as the age, weight and general condition of the patient, the condition that is being treated and the route by which the substance is being administered.

In summary, germination-deficient lysed suspension of B. subtilis can be administered without any danger associated with live bacteria generation but with all associated benefits and the biological effects induced by the content of the beneficiary microorganism colonies.

### DESCRIPTION OF DRAWINGS

The drawings are provided as a reference to possible embodiments and are not intended and should not limit the scope of the invention.

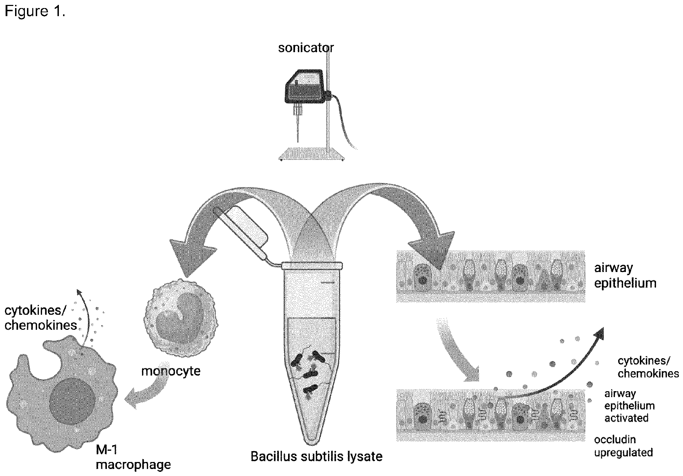

Figure 1. shows a conceptual design of the invention and the flowchart of the method application.

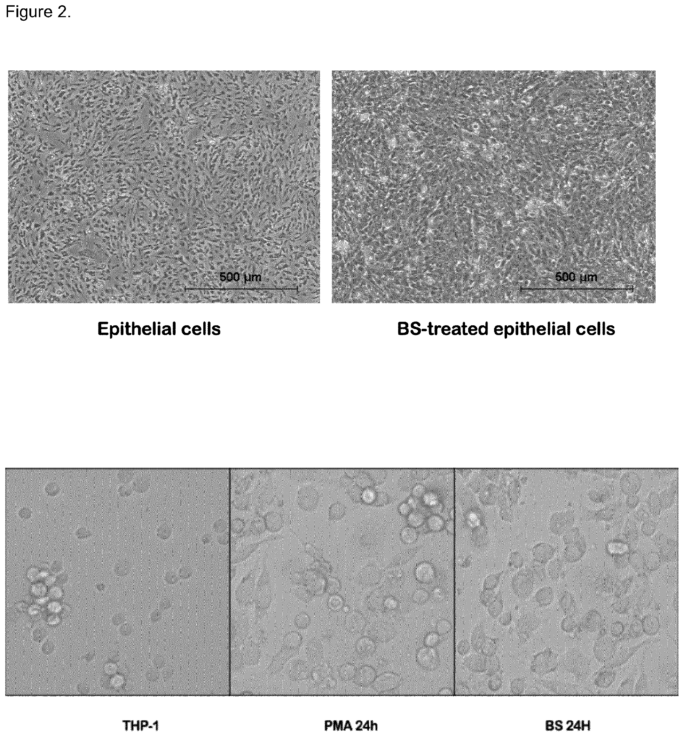

Figure 2. shows the morphology of bronchial epithelial cultures Beas-2B (upper panel) and Bacillus subtilis (BS) lysate-exposed human monocyte cell culture TH-1 (lower panel).
Figure 3. shows the occludin content in Bacillus subtilis lysate-treated bronchial epithelium cells in comparison to the control cells (upper left graph); the levels of secreted cytokines by the Bacillus subtilis lysate-treated bronchial epithelium Beas-2B in comparison to the control cells (other graphs).
Figure 4. shows the size and shape characteristics of TH-1 monocyte-derived macrophages after PMA and Bacillus subtilis lysate treatment (upper panel); the levels of secreted cytokines by the TH-1 monocyte-derived macrophages after PMA and Bacillus subtilis lysate treatment (other graphs).

### DETAILED DESCRIPTION OF INVENTION

### Process and steps of the method

The discovery pertains directly to the potential of B. subtilis lysate to act as a booster of innate immunity via induction of monocyte maturation and, subsequently, activate adaptive immunity and modulate epithelial reactivity in the human respiratory tract. The object of the invention described herein is a method for the induction of immune reaction in human bronchial epithelium and monocytes *in vitro* by using Bacillus subtilis lysate.

**The usage of the Bacillus subtilis lysate as an immunomodulator in bronchial epithelium cultures *in vitro.*** The treatment of epithelium by Bacillus subtilis possesses several key features and is demonstrated in this stepwise method:
1) the lysate is produced by exposure of the bacterial suspension to ultrasound as described elsewhere (31). The current method requires 5 min of sonication, resulting in the loss of viable bacteria and the release of DNA. The resulting lysates should be directly added the cell culture medium for cell biology experiments as described below;
2) a resulting lysate is used unmanipulated and contains the entire content of lysed 10⁸/mL of B. *subtilis* in 0.9% saline suspension;

1) Lysate does not reduce the exposed cell culture viability and is not cytotoxic;
2) Lysate doesn't interfere with cell attachment and proliferation for at least 96-hour period;
3) Procedures are performed in sterile conditions. A cell suspension is prepared via thawing the cells if they are frozen; centrifuging the cells to pellet them; discarding the supernatant, and resuspending the cells in a suitable growth medium; counting the cells using a hemacytometer or automated cell counter, and adjusting the cell density to the desired concentration; transferring the cell suspension to a sterile culture vessel; incubating cells at the appropriate temperature, humidity, and CO₂ level; until cells reach the desired density or confluency;
4) Passaged cells are seeded at a desired cell density in a specific cell culture medium supplemented with, but not limited to, fetal bovine serum (FBS)/fetal calf serum (FCS) and antibiotics/antimycotics. Next, the incubation should follow at 37°C, 21 % O₂, and 5% CO₂;
5) The effects described hereby were proven using a human non-tumorigenic airway epithelium cell line BEAS-2B. The cells were cultivated for a defined period of time, followed by the CCK-8 Assay testing (Dojindo). This method estimates cell viability by measuring the amount of chromogenic substrate generated by metabolically active cells. The absorbance is a direct measure of the amount of metabolized dye and is proportional to the number of viable cells. Therefore, higher absorbance indicates higher cell viability and vice versa. These results are determined by a spectrophotometer (absorbance);
6) The cell culture media/supernatants are then collected for the secretome analysis;
7) Bacillus subtilis lysate-treated cells are further lysed, and protein-containing lysates or RNA content is recovered as the manufacturer instructs. The resulting lysates and genetic material were subjected for the RNA/DNA/protein content and quality analysis;
8) The enzyme-linked immunoassays (ELISAs) were performed in the media of the cells treated with Bacillus subtilis lysate by adding primary antibodies targeting proteins of interest and other reagents according to the manufacturer's instructions. Ready-to-use ELISA kits for detecting various interleukins (IL)6, IL8, MCP1, and others were used according to the manufacturer's instructions.

### Experimental results

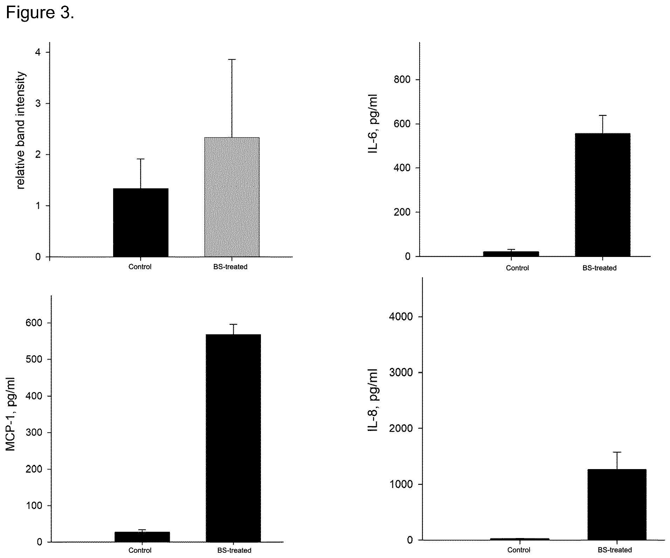

The Bacillus subtilis lysate-treated epithelium exhibited stable viability readings based on the CCK-8 test results, indicating good proliferation of cells over time and increasingly stable barrier function as indicated by the level of epithelial barrier protein occludin in the bronchial epithelium Beas-2B cell lysates (Figure 3).

Secretome content, namely inflammatory mediators IL6, IL8, and MCP1, was significantly upregulated in Bacillus subtilis lysate-treated epithelial cells in comparison to the control cells. The shifts detected indicate that the bronchial epithelial cells, as some other epithelial cell types (32), may form stronger monolayer cultures under treatment with Bacillus subtilis lysate. An increase in secreted cytokine content suggests that bronchial epithelium, as well as intestinal epithelium (33), may react to non-pathogenic Bacillus subtilis lysate with an induced cytokine secretion.

**The usage of the Bacillus subtilis lysate as a macrophage maturation-inducing agent *in vitro.*** The treatment of human monocytes by Bacillus subtilis possesses several key features:
1) the lysate described above should not reduce the viability of exposed monocytes and is not cytotoxic;
2) Monocytes can adhere and proliferate for at least 96 hours;
3) Procedures are performed in sterile conditions. The cells used were THP-1, i.e., a monocyte line isolated from peripheral blood from an acute monocytic leukemia patient. Cell culture is suspension type, and floating cells are viable. When maturing towards macrophages, monocytes attach, flatten, and change their shape. A cell suspension is prepared via thawing the cells if they are frozen, centrifuging the cells to pellet them, discarding the supernatant, resuspending the cells in a suitable growth medium, counting the cells using a hemacytometer or automated cell counter, and adjusting the cell density to the desired concentration; transferring the cell suspension to a sterile culture vessel; incubating cells at the appropriate temperature, humidity, and CO₂ level; until cells reach the desired density.
4) Passaged cells are seeded at a desired cell density in a cell culture medium supplemented with, but not limited to, fetal bovine serum (FBS)/fetal calf serum (FCS), and antibiotics/antimycotics. Next, the incubation should follow at 37°C, 21 % O₂, and 5% CO₂.
9) Cell viability is determined on the confluent cells layer by adding a viability indicator to the cell culture medium according to the manufacturer's instructions. Monocytes were cultivated for a defined period of time, followed by the CCK-8 Assay testing (Dojindo). This method estimates cell viability by measuring the amount of chromogenic substrate generated by metabolically active cells. The absorbance is a direct measure of the amount of metabolized dye and is proportional to the number of viable cells. Therefore, higher absorbance indicates higher cell viability and vice versa. These results are determined by spectrophotometry (absorbance).
10)The positive control cell differentiation was induced via a 12h exposure to 100 ng/ml phorbol 12-myristate 13-acetate (PMA, Sigma-Aldrich) in DMSO.
5) The shape and size of the treated cells were monitored and documented.
6) The cell culture media/supernatants are then collected for the secretome analysis.
7) Bacillus subtilis lysate-treated cells are further lysed, and protein-containing lysates or RNA content is recovered as the manufacturer instructs. The resulting lysates and genetic material need testing for RNA/DNA/protein content and quality and are subjected to the analysis.
8) The cell culture medium/supernatants should be collected and the secreted molecules (biomarkers) quantified. Ready-to-use ELISA kits detecting various interleukins, cytokines and chemokines, like (IL)6, IL8, MCP1, and others, should be used according to the manufacturer's instructions.

### Experimental results

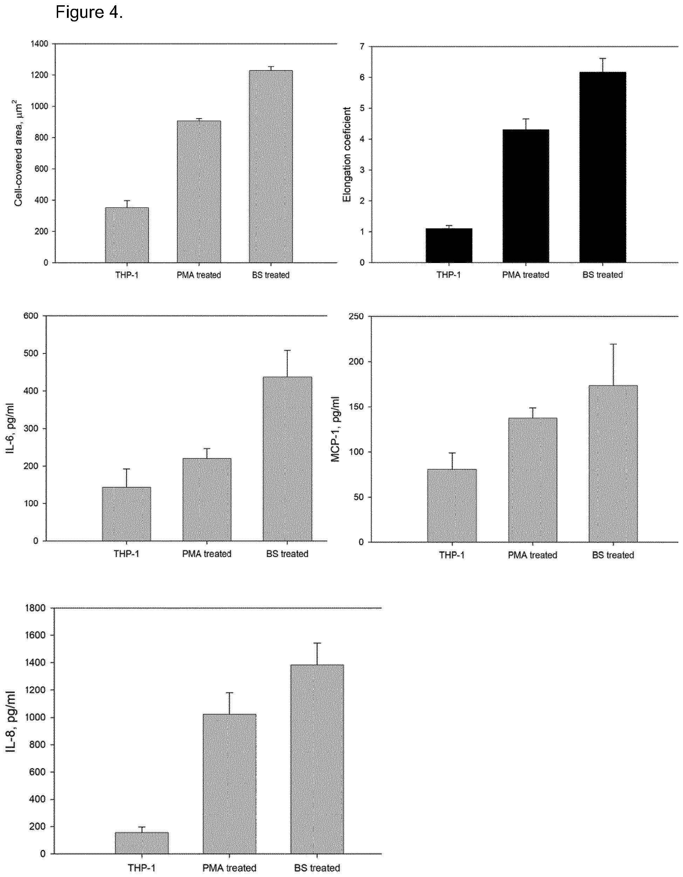

The assessment of Bacillus subtilis lysate-treated monocytes revealed no viability changes but rather significant macrophage phenotype induction, i.e., the change in cell size and shape and also secreted cytokines and chemokines in comparison to untreated cells. We have also compared the macrophage phenotype induced by Bacillus subtilis lysate to PMA treatment. PMA is a known and widely used inducer of monocyte maturation and macrophage differentiation. It is also toxic and unphysiological. The Bacillus subtilis-induced phenotype is presented in Figure 4. Secreted cytokine content was also compatible with the M-1 type macrophage phenotype (34). Production of Th1-type response cytokines and induction of macrophage M1 polarization is a known phenomenon, although M2-type induction by different components of Bacillus subtilis is also widely described. This finding provides significant insights for the use of probiotics with immunostimulatory effects. Overall, the changes documented hereby prove the efficient and physiological method for macrophage maturation induction exerted by Bacillus subtilis lysate.

### Embodiments

The presented method implemented the following procedure described in the Method application, which provides an embodiment scenario of epithelial cells and monocyte response to probiotic treatment in vitro. The lysate of Bacillus subtilis yielding 10⁸/mL of lysed non-viable B. *subtilis* in 0.9% saline provides an effective, cheap, and physiological tool for immunostimulation of human epithelial cells and monocytes in vitro when such stimulation is needed. The proposed method allows a broad selection of cell lines and tissue types for the induction of immunostimulation, going beyond human lung-derived cell lines. This may include, but is not limited to:
- airway epithelial cell types (like 16HBE14o-, Calu3, primary cells, iPSC-derived etc.)
- alveolar epithelial cells type I and II (A549, NCI-H292, primary cells, iPSC derived etc.)
- endothelial cells (HUVEC, lung microvascular endothelium, etc.)
- skin cells (HaCaT, etc.)
- immune cells (THP-1, RAW 264.7 cells (murine macrophage cells) etc.);
- cancer cells (SH-SY5Y cells, NRK-52E cells and many other).

Assays described herein may include (but also is not limited to)
- viability assays , like MTT, CCK8, Alamar blue, Calcein AM, Live/Dead kit, etc.

Quantification of the secreted biomarkers include, but is not limited to ELISA, Luminex or PCR assays.

Obtained extracted proteins may be tested by means of Western Blot, Luminex, but is not limited to.

### Applications of the method

The presented method represents an efficient, cheap, simple and fast method to be used in in vitro by scientists and enterprises developing methods for cell maturation, immune response modulation and/or activation. Applications thereof are intended to be used for obtaining physiologically stimulated epithelium or monocyte-derived macrophages. In more detail, applications include:
∘ Differentiation of monocyte-derived macrophages *ex vivo* and *in vitro.* Namely, inducing Th1 type immune response, which is potentially useful to treat, prevent, or ameliorate allergic reactions in the respiratory tract. Allergic reactions are certain hypersensitivity reactions exerted in reaction to allergens and include allergic rhinitis, bronchial asthma and allergic bronchitis. Allergens' list includes pollen, dust, fungal spores, drugs etc.
∘ Induction of mild inflammatory activation within airway epithelium to produce IL6, IL8, and MCP-1, which is important to generate an immune response and inflammatory cell recruitment. Production of IL6 and IL8 is crucial to maintain Th1 responses and to produce other pro-inflammatory antibodies. MCP-1 is involved in the additional recruitment of monocytes, also leading to the augmented response.

### References

1. Asrat S, Davis KM, Isberg RR. Modulation of the host innate immune and inflammatory response by translocated bacterial proteins. Cell Microbiol. 2015;17(6):785-795.
2. Koopman N, van Leeuwen P, Brul S, Seppen J. History of fecal transplantation; camel feces contains limited amounts of Bacillus subtilis spores and likely has no traditional role in the treatment of dysentery. PLoS One. 2022;17(8):e0272607.
3. Tran, D.M., Tran, T.T., Phung, T.T.B. et al. Nasal-spraying Bacillus spores as an effective symptomatic treatment for children with acute respiratory syncytial virus infection. Sci Rep 12, 12402 (2022).
4. Cao XY, Aimaier R, Yang J, Yang J, Chen ZY, Zhao JJ et al. Effect of bacillus subtilis strain Z15 secondary metabolites on immune function in mice. BMC Genomics. 2023;24(1):273.
5. Li G, Tong Y, Xiao Y, Huang S, Zhao T, Xia X. Probiotic Bacillus subtilis contributes to the modulation of gut microbiota and blood metabolic profile of hosts. Comp Biochem Physiol C Toxicol Pharmacol. 2023;272:109712.
6. Caulier, S., Nannan, C., Gillis, A., Licciardi, F., Bragard, C., & Mahillon, J. (2019). Overview of the Antimicrobial Compounds Produced by Members of the Bacillus subtilis Group. Frontiers in Microbiology, 10, 435128.
7. Knapen, M., Drummen, N., Smit, E., Vermeer, C., Theuwissen, E. (2013). Three-year low-dose menaquinone-7 supplementation helps decrease bone loss in healthy postmenopausal women. Osteoporos Int. 24, 2499-2507.
8. Geleijnse, J. M., Vermeer, C., Grobbee, D. E., Schurgers, L. J., Knapen, M. H. J., van der Meer, I. M., et al. (2004). Dietary intake of menaquinone is associated with a reduced risk of coronary heart disease: the Rotterdam Study. J. Nutr. 134, 3100-3105.
9. Meng, F., Chen, R., Zhu, X., Lu, Y., Nie, T., Lu, F., et al. (2018). Newly Effective Milk-Clotting Enzyme from Bacillus subtilis and Its Application in Cheese Making. J. Agric. Food Chem. 66, 6162-6169.
10.Bureros, K. J. C., Dizon, E. I., Israel, K. A. C., Abanto, O. D., and Tambalo, F. Z. (2020). Physicochemical and sensory properties of carabeef treated with Bacillus subtilis (Ehrenberg) Cohn protease as meat tenderizer. J. Food Sci. Technol. 57, 310-318
11.Cheng, Y. H., Zhang, N., Han, J. C., Chang, C. W., Hsiao, F. S. H., and Yu, Y. H. (2018). Optimization of surfactin production from Bacillus subtilis in fermentation and its effects on Clostridium perfringens-induced necrotic enteritis and growth performance in broilers. J. Anim. Physiol. Anim. Nutr. 102, 1232-1244.
12.Ongena, M., and Jacques, P. (2008). Bacillus lipopeptides: versatile weapons for plant disease biocontrol. Trends Microbiol. 16, 115-125.
13.Jacques, P. (2011). Surfactin and other lipopeptides from Bacillus spp., in Biosurfactants: From Genes to Applications, eds Soberón-Chávez G. Berlin, Heidelberg: Springer; p. 57-91.
14.Jemil, N., Ben Ayed, H., Manresa, A., Nasri, M., and Hmidet, N. (2017a). Antioxidant properties, antimicrobial and anti-adhesive activities of DCS1 lipopeptides from Bacillus methylotrophicus DCS1. BMC Microbiol. 17, 144.
15. Kim, S. D., Park, S. K., Cho, J. Y., Park, H. J., Lim, J. H., Yun, H. I., et al. (2006). Surfactin C inhibits platelet aggregation. J. Pharm. Pharmacol. 58, 867-870.
16. Park, S. Y., and Kim, Y. (2009). Surfactin inhibits immunostimulatory function of macrophages through blocking NK-kappaB, MAPK and Akt pathway. Int. Immunopharmacol. 9, 886-893
17.Vassaux, A., Rannou, M., Peers, S., Daboudet, T., Jacques, P., and Coutte, F. (2021). Impact of the purification process on the spray-drying performances of the three families of lipopeptide biosurfactant produced by Bacillus subtilis. Front. Bioeng. Biotechnol. 9, 815337
18.Vollenbroich, D., Ozel, M., Vater, J., Kamp, R. M., and Pauli, G. (1997). Mechanism of inactivation of enveloped viruses by the biosurfactant surfactin from Bacillus subtilis. Biol. J. Int. Assoc. Biol. Stand. 25, 289-297.
19.Kiran S., Priyadharsini S., Sajayan A., Priyadharsini G. B., Poulose N., Selvin, J. (2017). Production of lipopeptide biosurfactant by a marine Nesterenkonia sp. and its application in food industry. Front. Microbiol. 8, 1138.
20.Ohadi, M., Forootanfar, H., Rahimi, H. R., Jafari, E., Shakibaie, M., Eslaminejad, T., et al. (2017). Antioxidant potential and wound healing activity of biosurfactant produced by Acinetobacter junii B6. Curr. Pharm. Biotechnol. 18, 900-908.
21.Kanlayavattanakul, M., and Lourith, N. (2010). Lipopeptides in cosmetics. Int. J. Cosmet. Sci. 32, 1-8.
22.Shahbazi, R., Yasavoli-Sharahi, H., Alsadi, N., Ismail, N. & Matar, C. Probiotics in treatment of viral respiratory infections and neuroinflammatory disorders. Molecules 25, 4891 (2020).
23. Lehtoranta, L., Pitkäranta, A. & Korpela, R. Probiotics in respiratory virus infections. Eur. J. Clin. Microbiol. Infect. Dis. 33, 1289-1302 (2014).
24.Starosila D, Rybalko S, Varbanetz L, Ivanskaya N, Sorokulova I. Anti-influenza Activity of a Bacillus subtilis Probiotic Strain. Antimicrob Agents Chemother. 2017 Jun 27;61(7):e00539-17.
25.Salminen, S. et al. The international scientific association of probiotics and prebiotics (ISAPP) consensus statement on the definition and scope of postbiotics. Nat. Rev. Gastroenterol. Hepatol. 18, 649-667 (2021).
26. Kumpu, M. et al. Milk containing probiotic Lactobacillus rhamnosus GG and respiratory illness in children: A randomized, double-blind, placebo-controlled trial. Eur. J. Clin. Nutr. 66, 1020-1023 (2012).
27. Luoto, R. et al. Prebiotic and probiotic supplementation prevents rhinovirus infections in preterm infants: A randomized, placebo-controlled trial. J. Allergy Clin. Immunol. 133, 405-413 (2014).
28. Liu, S., Hu, P., Du, X., Zhou, T. & Pei, X. Lactobacillus rhamnosus GG supplementation for preventing respiratory infections in children: A meta-analysis of randomized, placebo-controlled trials. Indian Pediatr. 50, 377-381 (2013).
29.Anaya-Loyola, M. A. et al. Bacillus coagulans GBI-30, 6068 decreases upper respiratory and gastrointestinal tract symptoms in healthy Mexican scholar-aged children by modulating immune-related proteins. Food Res. Int. 125, 108567 (2019).
30. Kawase, M. et al. Heat-killed Lactobacillus gasseri TMC0356 protects mice against influenza virus infection by stimulating gut and respiratory immune responses. FEMS Immunol. Med. Microbiol. 64, 280-288 (2012).
31. Fykse, E. M., Olsen, J. S., & Skogan, G. (2003). Application of sonication to release DNA from Bacillus cereus for quantitative detection by real-time PCR. Journal of Microbiological Methods, 55(1), 1-10.
32. Gu MJ, Song SK, Park SM, Lee IK, Yun CH. Bacillus subtilis Protects Porcine Intestinal Barrier from Deoxynivalenol via Improved Zonula Occludens-1 Expression. Asian-Australas J Anim Sci. 2014;27(4):580-6.
33. Hosoi T, Hirose R, Saegusa S, Ametani A, Kiuchi K, Kaminogawa S. Cytokine responses of human intestinal epithelial-like Caco-2 cells to the nonpathogenic bacterium Bacillus subtilis (natto). Int J Food Microbiol. 2003;82(3):255-64.
34.Tobita, K., & Meguro, R. (2022). Bacillus subtilis BN strain promotes Th1 response via Toll-like receptor 2 in polarized mouse M1 macrophage. Journal of Food Biochemistry, 46(2), e14046.

## Claims

1. A composition for in vitro application, comprising lysate of Bacillus subtilis in an amount effective to stimulate or enhance epithelial cells inflammatory mode.

2. A composition, according to claim 1, in which the lysate delivers either (a) an immunostimulatory effect, or (b) a barrier-enhancing effect, or (c) any combination of these two.

3. A composition, according to claim 1 or 2, in which the lysate is germination-deficient.

4. A composition, according to claim 1, 2, or 3, in which the lysate functions as a cell differentiation inductor or activator of antigen-presenting cells.

5. A composition, according to claim 1, 2, 3 or 4, in a pharmaceutically acceptable carrier or diluent for oral, nasal, respiratory, pulmonary topical, and buccal (sublingual) administration.

6. A method of boosting an immune response; which comprises administering a lysate of Bacillus subtilis to humans.

7. A method of maturing monocytic cells in vitro which comprises treating the cells with the lysate of Bacillus subtilis.
